# EUROPEAN PATENT APPLICATION

(11) **EP 2 591 739 A1**
(43) Date of publication of application: **15.05.2013**
(21) Application number: 11189054.7
(22) Date of filing: 14.11.2011
(51) Int. Cl.: A61B 17/70

(54) **Polyaxial bone anchoring device**

(71) Applicant: Biedermann Technologies GmbH & Co. KG, 78166 Donaueschingen (DE)
(72) Inventor: Biedermann, Lutz, 78048 VS-Villingen (DE); Matthis, Wilfried, 79367 Weisweil (DE); Fischer, Bernd, 78199 Bräunlingen (DE)
(74) Representative: Prüfer & Partner GbR European Patent Attorneys

(57) **Abstract**

A polyaxial bone anchoring device is provided including a bone anchoring element (1) having a shank (2) for anchoring in the bone and a head (3); a head receiving part (5) having a first end (5a) and an open second end (5b) with a bounding edge and a hollow interior portion (54) in communication with the open second end for receiving the head (3) therein, the head receiving part (5) being flexible so as to allow clamping of the head; a locking ring (6) mounted around the head receiving part, the locking ring having a rod receiving portion (63, 64); wherein the head (3) is pivotable in the head receiving part and can be locked at an angle by compressing the head receiving portion by means of the locking ring (6); a cap (8) configured to cover the rod and to engage the head receiving part, a locking element (9) extending through the cap and configured to engage the rod;
wherein the cap (8) and the locking ring (6) are rotatable with respect to the head receiving part (5) when the rod is inserted and upon tightening of the locking element the rod and the bone anchoring element are locked with respect to each other.

## Description

The invention relates to a polyaxial bone anchoring device that includes a bone anchoring element for anchoring in the bone, a head receiving part for receiving a head of the bone anchoring element, a locking ring mounted around the head receiving part for coupling a stabilization rod to the head receiving part and the bone anchoring element, a cap for securing the rod and a locking element for locking the whole assembly. Upon connection of the cap to the head receiving part, the sub-assembly consisting of the locking ring, the rod and the cap is rotatable with respect to the head receiving part. The locking element exerts pressure onto the rod to move the locking ring in a position to lock the head in the head receiving part and to fix the rod simultaneously. The polyaxial bone anchoring device is particularly suitable for providing an enlarged pivot angle for the bone anchoring element.

A polyaxial bone anchoring device with an enlarged pivot angle is described in US 6,736,820. This bone anchoring device comprises a bone screw and a receiving part with a seat for the head of the bone screw. The screw member can be pivoted to at least one side by an enlarged angle, because the edge of the free end of the receiving part is of asymmetric construction.

Another polyaxial bone anchor is described in US 2005/0080415 A1. This bone anchor has a body member having a U-shaped channel for receiving the rod and a compressible recess for receiving a head of the anchor member such that the anchor member can initially polyaxially angulate with respect to the body member and further has a collar slideably disposed about the body member and capable of compressing the recess around the head. The lower bounding edge of the body member may include a counter-sunk region to permit increased angulation, when the anchor member is oriented toward the counter-sunk region.

US 2009/0149887 A1 describes an apparatus for connecting a bone anchor to a support rod that includes a connector body and a cap.

It is the object of the invention to provide a polyaxial bone anchoring device that has a low profile and in particular that permits the bone anchoring element to pivot at an enlarged angle in a specific direction.

The object is solved by a polyaxial bone anchoring device according to claim 1. Further developments are given in the dependent claims.

The polyaxial bone anchoring device makes use of a cap and a locking element received in the cap for securing and fixing the rod and the head . Therefore, a problem of splaying of portions of a receiving part when tightening a locking element as it may be the case in conventional receiving parts does not occur. Furthermore, with the cap, the bone anchoring device has a low profile in axial or height direction of the bone anchoring element.

With the polyaxial bone anchoring device a modular screw system can be provided. The modular system includes the head receiving part preassembled with the locking ring and the cap pre-assembled with the locking element and a set of bone anchoring elements having different shanks. By means of this, various shanks with different diameter, thread form or other different features can be combined with a head receiving part and locking ring according to the requirements in a particular clinical situation. In a further modular system, different head receiving parts with different bounding edges for allowing different maximum pivot angles are provided, that can be assembled with a locking ring and a cap with locking element and various shanks. Therefore, the surgeon has a substantial choice of implants. By the modularity, the costs of stock-holding can be decreased.

When the head receiving part has a free bounding edge that is configured to permit the anchoring element to pivot at a larger pivot angle at a first location of the bounding edge than at a second location of the bounding edge, the pivot angle can be selected in situ within a range of 360° around the central axis of the head receiving part. The maximum pivot angle of the bone anchoring element relative to the head receiving part is equal to or greater than 45° measured from the straight position. The orientation of the enlarged pivot angle can be selected, for example, in a plane including the rod axis in the shank axis or at 90° with respect to the rod axis or at any other angle. This renders the bone anchoring device particularly suitable for the application of lateral mass fixation, for example, for the cervical spine.

The design of the bone anchoring device allows to further reduce the dimensions in terms of height as well as in terms of diameter, which makes it particularly suitable for applications, where small-sized anchoring devices are required, such as in the field of cervical spine surgery or pediatric applications, trauma and minimally open applications for bone surgery.

Further features and advantages will become apparent from the description of embodiments by means of accompanying drawings.

In the drawings:
- Fig. 1:: shows an exploded perspective view of the polyaxial bone anchoring device according to a first embodiment.
- Fig. 2:: shows a perspective view of the polyaxial bone anchoring device of Fig. 1 in an assembled state.
- Fig. 3:: shows a cross-sectional view of the polyaxial bone anchoring device, the section taken perpendicular to the rod axis, in a first pivot position of the bone anchoring element.
- Fig. 4:: shows a cross-sectional view of the polyaxial bone anchoring device, the section taken perpendicular to the rod axis, in a second pivot position of the bone anchoring element.
- Fig. 5:: shows an enlarged view of a portion of Fig. 4.
- Fig. 6:: shows a perspective view of the head receiving part of the polyaxial bone anchoring device according to the first embodiment.
- Fig. 7:: shows a cross-sectional view of the head receiving part shown in Fig. 6.
- Fig. 8:: shows a perspective view of a locking ring of the polyaxial bone anchoring device according to the first embodiment.
- Fig. 9:: shows a cross-sectional view of the locking ring, the section taken along line B-B in Fig. 8.
- Fig. 10:: shows a perspective view from the top of the cap of the polyaxial bone anchoring device according to the first embodiment.
- Fig. 11:: shows a perspective view from below of the cap of the bone anchoring device.
- Fig. 12:: shows a side view of the cap.
- Fig. 13:: shows another side view of the cap, rotated by 90°.
- Fig. 14:: shows a top view of the cap.
- Fig. 15:: shows a cross-sectional view of the cap, the sections taken along lines A-A in Fig. 14.
- Fig. 16:: shows an enlarged view of a portion of Fig. 15.
- Figs. 17 to 21:: shows steps of assembly and use of the polyaxial bone anchoring device according to the first embodiment.
- Fig. 22:: shows an exploded perspective view of the polyaxial bone anchoring device according to a second embodiment.
- Fig. 23:: shows a perspective view of the polyaxial bone anchoring device of Fig. 22 in an assembled state.
- Fig. 24:: shows a cross-sectional view of the polyaxial bone anchoring device according to the second embodiment, the section taken perpendicular to the rod axis, in a first pivot position of the bone anchoring element.
- Fig. 25:: shows a cross-sectional view of the polyaxial bone anchoring device of the second embodiment, the section taken perpendicular to the rod axis in a second pivot position of the bone anchoring element.
- Fig. 26:: shows an enlarged view of a portion of Fig. 25.
- Fig. 27:: shows a perspective view from the top of the head receiving part of the polyaxial bone anchoring device according to the second embodiment.
- Fig. 28:: shows a cross-sectional view of the head receiving part of Fig. 27.
- Fig. 29:: shows a perspective view of the locking ring of the polyaxial bone anchoring device according to the second embodiment.
- Fig. 30:: shows a cross-sectional view of the locking ring, the section taken along line D-D in Fig. 29.
- Fig. 31:: shows a perspective view from the top of the cap of the polyaxial bone anchoring device according to the second embodiment.
- Fig. 32:: shows a perspective view of the cap of the polyaxial bone anchoring device according to the second embodiment from below.
- Fig. 33:: shows a side view of the cap.
- Fig. 34:: shows another side view of the cap, rotated by 90°.
- Fig. 35:: shows a top view of the cap.
- Fig. 36:: shows a cross-sectional view of the cap, the section taken along line C-C in Fig. 35.
- Fig. 37:: shows an enlarged view of a part of Fig. 36.
- Fig. 38:: shows a perspective view from the top of a head receiving part of the polyaxial bone anchoring device according to a third embodiment.
- Fig. 39:: shows a cross-sectional view of the head receiving part shown in Fig. 38.
- Fig. 40:: shows a top view of the head receiving part of Fig. 38.

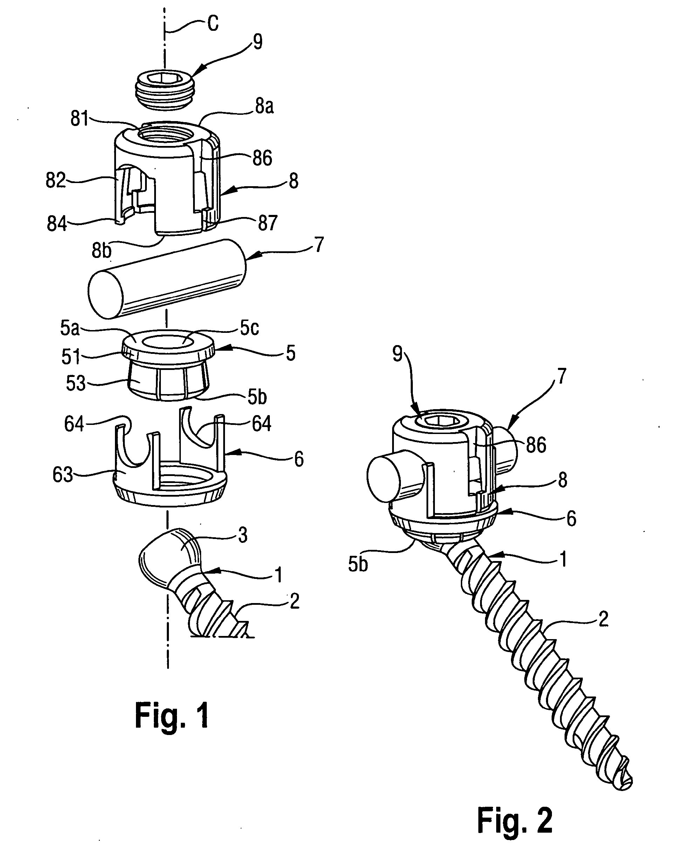

As shown in Figs. 1 to 5, the polyaxial bone anchoring device according to a first embodiment comprises a bone anchoring element 1 in the form of a bone screw having a shank 2 with a threaded section and a spherical segment-shaped head 3. The head 3 has a recess 4 for engagement with a tool. The bone anchoring device further includes a head receiving part 5 for receiving the head 3 of the bone anchoring element and a locking ring 6 for receiving a rod 7, for example a spinal stabilization rod, and for connecting the rod 7 to the bone anchoring element 1. In addition, the bone anchoring device comprises a cap 8 for securing the rod 7 and a locking element 9 in the form of a set screw for locking the rod and the head 3.

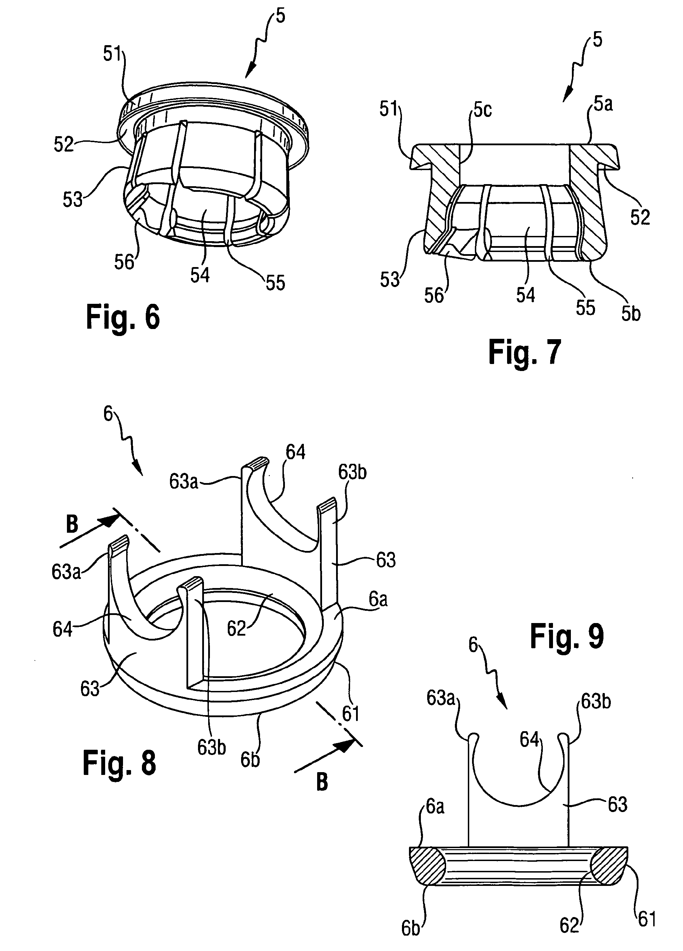

Referring in particular to Figs. 6 to 9, the head receiving part 5 has a first end 5a and an opposite second end 5b and a coaxial through hole 5c. Adjacent to the first end 5a is a substantially outwardly extending rim portion 51. The rim portion 51 has on its side facing away from the first end 5a a circumferentially extending undercut portion 52. The undercut portion 52 is formed by a surface that is inclined outwards and towards the second end 5b. The undercut portion 52 serves for engagement with a portion of the cap 8.

The head receiving part 5 further includes a conically-shaped outer surface portion 53 that widens towards the second end 5b. The maximum outer diameter of the head receiving part at the second end 5b is smaller than at the first end 5a. An internal hollow spherical section 54 forming an accommodation space for the spherical segment-shaped head 3 of the bone anchoring element 1 is formed in the head receiving part 5. The internal hollow spherical section 54 is configured to encompass the head of the bone anchoring element from the side covering a region including a largest diameter of the head 3.

A plurality of slits 55 are provided that are open to the second end 5b. The slits 55 extend substantially through the wall of the internal hollow portion 54 and render the head receiving part flexible in the region where the head 3 is received. By the size and number of the slits a desired elasticity is provided to the head receiving part. The elasticity of the head receiving part 5 is such that the head 3 of the anchoring element 1 can be inserted by expanding the head receiving part and can be clamped by compressing the head receiving part.

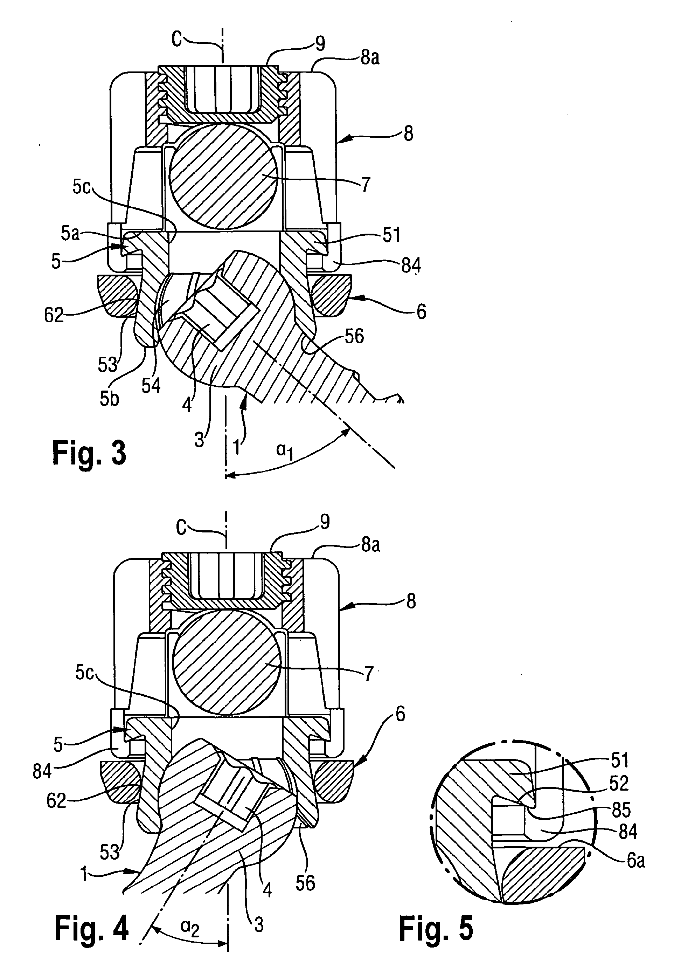

The edge bounding the second end 5b is asymmetric. In the embodiment shown, this is achieved by a counter-sunk or recessed area 56 provided in the wall of the internal hollow space 54. By means of this, the anchoring element can pivot at the position of the recessed are 56 to a larger pivot angle α₁ with respect to the straight position, when the anchor axis A of the anchoring element 1 is coaxial to the central axis C of the head receiving part 5 (Fig. 3) compared to a smaller pivot angle α₂ in the opposite direction (Fig. 4). The recessed area 56 defines the position of the enlarged pivot angle with respect to the locking ring 6 as described below.

The locking ring 6 is shown in more detail in Figs. 8 and 9. The locking ring 6 has a slightly conical outer surface portion 61 and at its inner side a curved internal surface portion 62. The curvature is directed to the center of the locking ring. The curved surface portion 62 can have a spherical curvature but other types of curvatures are also possible. The inner diameter of the locking ring is such that the locking 6 can slide along the outer conical surface portion 53 of the head receiving part 5, thereby compressing the head receiving part 5 in an increasing manner when sliding downwards.

On its side facing the rim portion 51 of the head receiving portion 5, the locking ring 6 comprises two projections 63 located diametrically opposite to each other. The projections 63 have such a height that they project up and away from the first end 5a of the head receiving part, when the locking ring 6 is mounted around the head receiving part 5, as depicted in Fig. 2. At their free ends the projections each have a recess 64, that has the shape of the segment of a circle including at least a portion with the greatest diameter. The diameter of the circular segment-shaped recess 64 corresponds substantially to the diameter of the rod 7. By means of the recess 64 each projection 63 comprises upstanding legs 63a, 63b that allow the rod 7 to be clicked between them.

The flexibility of the head receiving part 5 and the size of the head receiving part 5 at the open second end 5b allows to mount the locking ring 6 by assembling it from the second end 5b. When the locking ring is mounted onto the head receiving part 5, it is freely rotatable around the head receiving part 5. The dimensions of the parts can be designed such that the head 3 is preliminarily held in the head receiving part 5 by a slight friction force and the locking ring 6 is held by a friction force in a preliminary manner around the head receiving part.

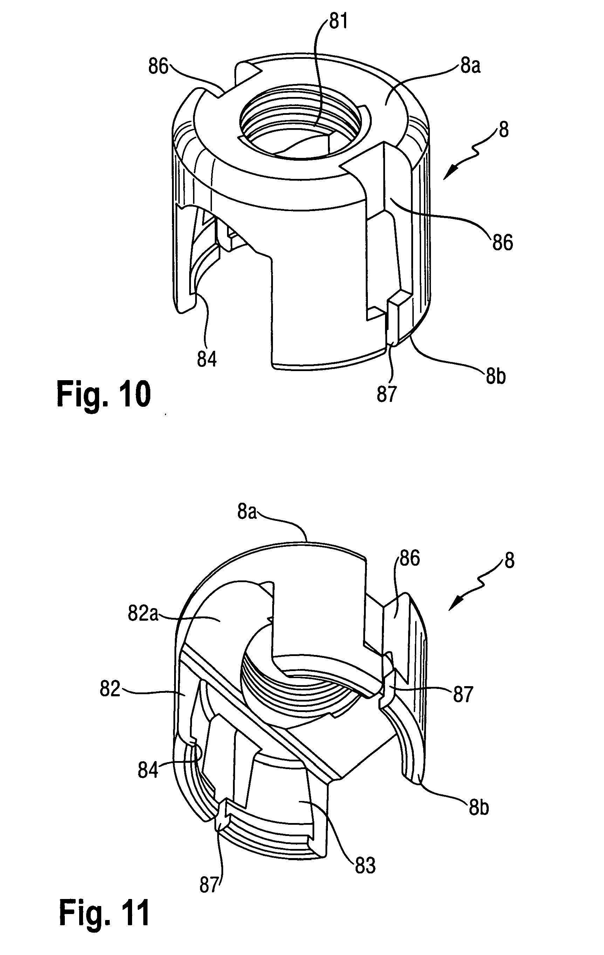

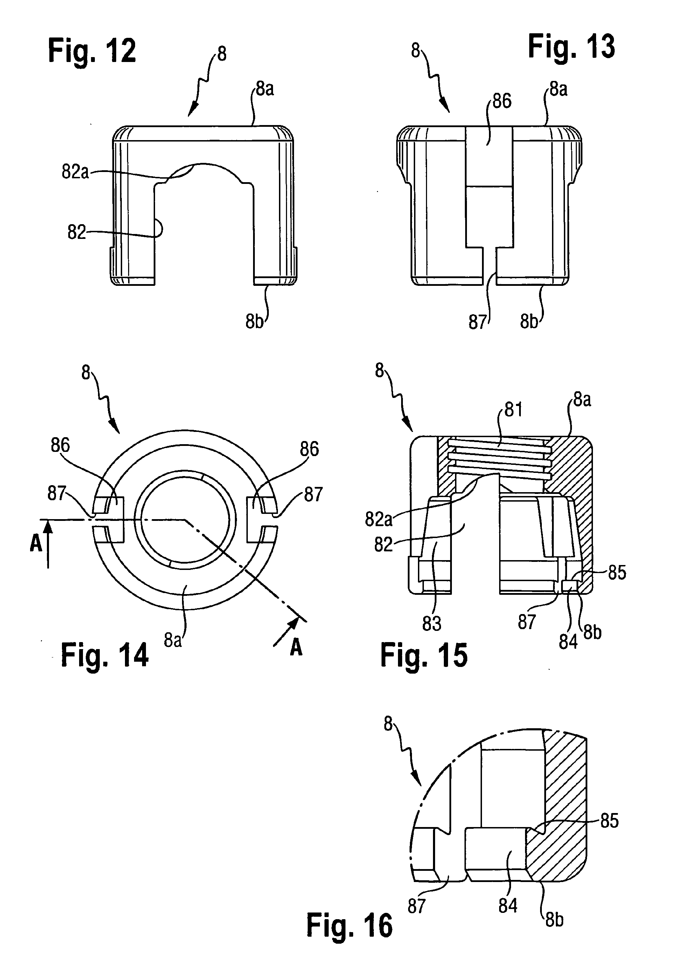

The cap will now be described with reference to Figs. 10 to 16. The cap 8 is a substantially cylindrical part with a first end 8a and a second end 8b. At the first end 8a, there is a coaxial threaded through hole 81 for receiving the locking element 9. At the second end 8b, there is a substantially cuboid-shaped recess 82, that continues in a cylindrical recess 82a with the cylinder axis standing perpendicular to the axis of the threaded through hole 81. The depth of the recess 82 and 82a is such that the rod 7 can be covered by the recess and in addition, the cap can be mounted to the head receiving part 5.

At its second end 8b, the cap further comprises a coaxial substantially conical recess 83. By means of this, the wall thickness of the cap decreases towards the second end. An inwardly projecting rim 84 is provided at the second end that has an undercut portion 85 with an inclined surface, wherein the inclination substantially corresponds to the inclination of the undercut portion 52 of the head receiving part 5. The size of the rim 84 with the undercut portion 85 is such that the rim 84 can engage the undercut portion 52 at the rim portion 51 of the head receiving part 5.

The cap further comprises two engagement portions 86 for holding, mounting or removal of the cap with a tool. The engagement portions 86 extend from the first end 8a to a distance from the second end 8b and are located each at 90° with respect to the rod receiving recesses 82, 82a. The engagement portions 86 are formed as recessed portions configured to be engaged with a tool. The wall thickness of the cap at the engagement portion 86 is reduced. At the positions of the engagement portion 86, the cap has a slit 87, respectively, which extends from the respective engagement portion to the second end 8b completely through the wall of the cap. By means of the slits 87, the wall portions of the cap are flexible to some extent so as to allow mounting of the cap to the head receiving part in such a way that the rim 84 of the cap snaps behind the rim 51 of the head receiving part 5.

The locking element 9 in the form of a set screw is configured to be screwed into the threaded through hole 81. Although the thread is shown to be a flat thread, any other thread form, such as a metric thread, can be used.

The head receiving part 5, the locking ring 6, the cap 8 and the locking element 9 as well as the bone anchoring element 1 are made of a bio-compatible material, for example, of titanium or stainless steel or of bio-compatible alloys, such as nickel-titanium alloys, for example Nitinol, or of a bio-compatible plastic material, such as, for example, polyether ether ketone (PEEK). The parts can be made all of the same or of different materials.

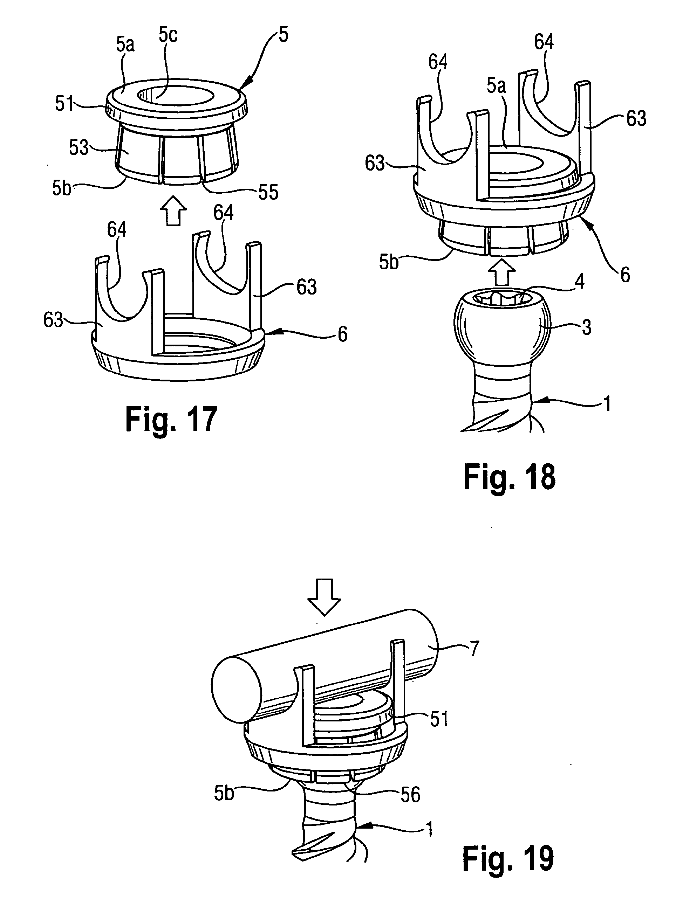

Referring to Figs. 17 to 21, steps of mounting and use of the polyaxial bone anchoring device will be explained. As shown in Fig. 17, the locking ring is mounted from the second end 5b to the head receiving part 5 such that, as shown in Fig. 18, the projections 63 for receiving the rod extend upwards from the first end 5a of the head receiving part 5. The head receiving part 5 and the locking ring 6 may be delivered in pre-assembled manner. In a second step shown in Fig. 18, the bone anchoring element 1 with a suitable shank 2 for a specific clinical application is mounted from the second end 5b to the assembly formed by the head receiving part 5 and the locking ring. The locking ring 6 is in its uppermost position where its first end 6a abuts against the rim portion 51 of the head receiving part 5. The head 3 is introduced into the internal hollow section 54 of the head receiving part. This is possible, because the head receiving part is flexible.

At this stage, the modularity of the bone anchoring device allows to combine a specific bone anchoring element with a specific shank with the head receiving part during or before surgery. When the head receiving part 5 and locking ring 6 are mounted together, they are still rotatable with respect to each other. Therefore, the position of head receiving part 5 with the recessed area 56 can be freely selected by rotating the head receiving part 5 with respect to the locking ring 6. Hence, by providing different head receiving parts with different predefined maximum pivot angles, which can be combined with a bone anchoring element having a specific shank, a suitable bone anchoring device can be selected and assembled easily.

The polyaxial bone anchoring device thus assembled is then inserted into the bone or a vertebra. In an alternative way of use, the bone anchoring element is first inserted into the bone or a vertebra and thereafter the combination of head receiving part 5 and locking ring 6 is mounted to the head 3 after implantation of the screw.

Then, as shown in Fig. 19, the rod 7 is inserted into the recess 64 provided at the projections 63. Because the recess 64 has a circular segment shape including more than a largest diameter, the rod can be clicked into the recesses so that it is preliminarily held in place therein. The whole assembly consisting of the rod 7 and the locking ring 6 is still rotatable with respect to the head receiving part 5.

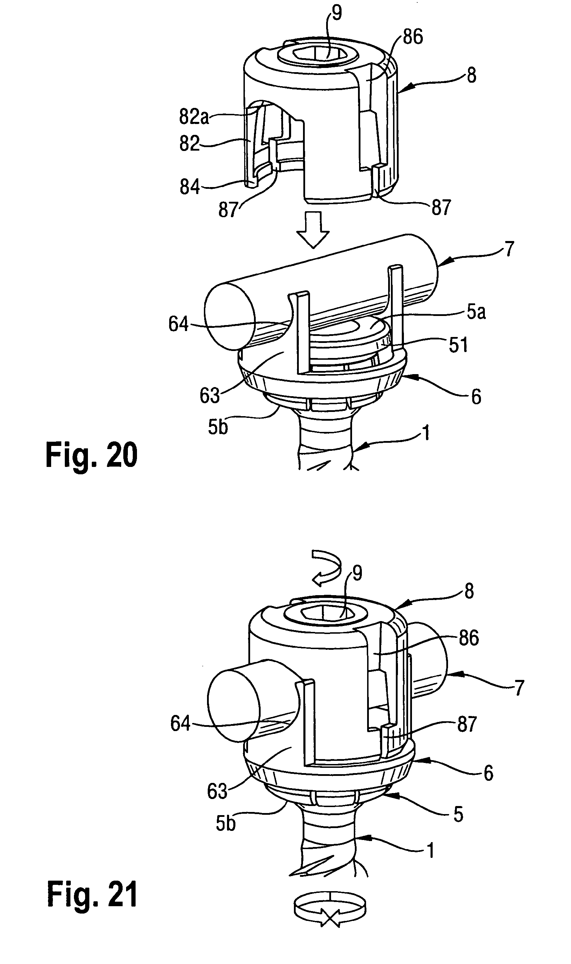

In a next step, as shown in Fig. 20, the cap 8 with the locking element 9 inserted therein is placed onto the assembly. Because the wall sections of the cap are slightly flexible, the cap can be clicked onto the head receiving part so that its rim portion 84 snaps behind the rim portion 51 of the head receiving part 5 and the undercut portions 85, 85 engage each other. This is shown in detail in Fig. 5.

Finally, as shown in Fig. 21, the locking element 9 in form of the set screw is tightened until its lower side presses onto the rod 7. As a consequence thereof, the rod 7 presses the locking ring 6 downward to compress the head receiving part 5. Final tightening of the locking element 9 locks the rod 7 and the head 3 simultaneously.

Referring to Figs. 3 to 5, the bone anchoring element can assume a position with a largest pivot angle α₁ with respect to the central axis C of the device when it is pivoted to the side including the recesses area 56 (Fig. 3). As shown in Fig. 4, the bone anchoring element can assume a second pivot position in the opposite direction or any other direction with a pivot angle α₂ being smaller than α₁.

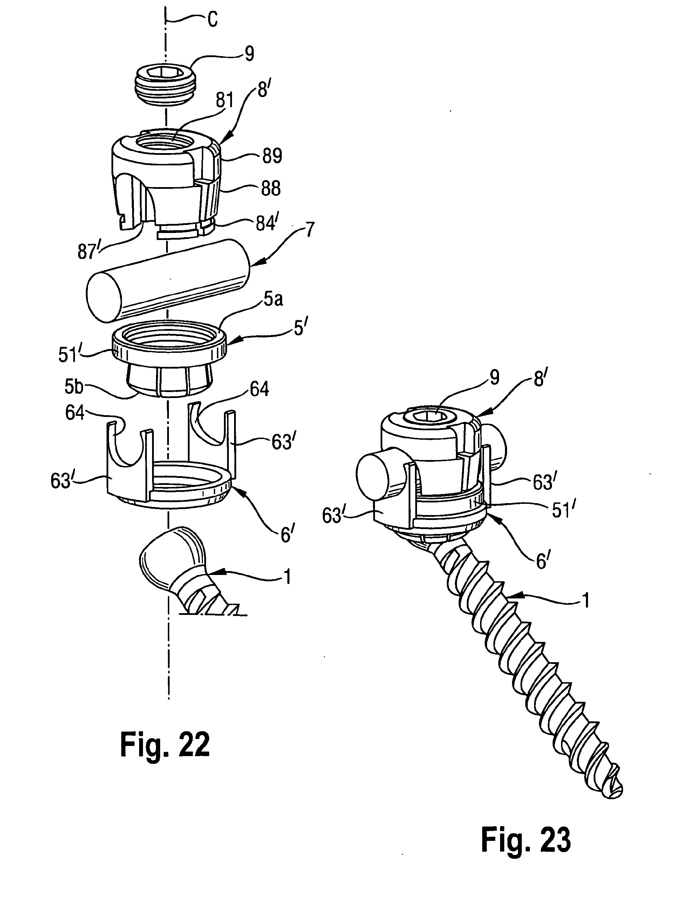

A second embodiment of a polyaxial bone anchoring device will be described with reference to Figs. 22 and 23. Parts and portions that are identical to the parts and portions of the first embodiment are designated with the same reference numerals and the description thereof shall not be repeated. The bone anchoring device according to the second embodiment differs from the bone anchoring device according to the first embodiment in the design of the head receiving part, the locking ring and the cap.

As shown in particular in Figs. 22 to 28 the head receiving part 5' comprises a rim 51' extending beyond the second end 5b seen in a radial direction. Adjacent to the first end 5a, a cylindrical recess 51a' is provided with an inner diameter larger than the outer diameter of the head receiving part at the second end 5b. Furthermore, a groove 51b' is provided with undercut portion 52' facing away from the first end 5a. The groove 51b' and the undercut portion 52' serve for engagement with a portion of the cap.

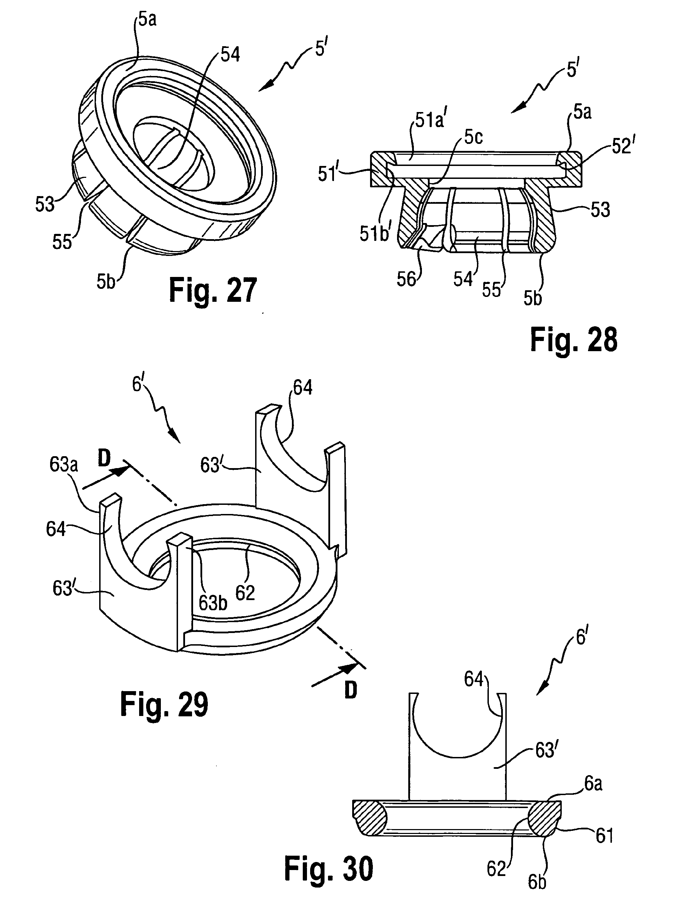

As shown in Figs. 29 and 30, the locking ring 6' has rod receiving projections that are placed at the outer-circumference of the locking ring. Therefore, the height of the projections 63 projecting above the head receiving part is smaller and the cap can be made shorter correspondingly so that the bone anchoring device as a whole has a lower profile in axial direction.

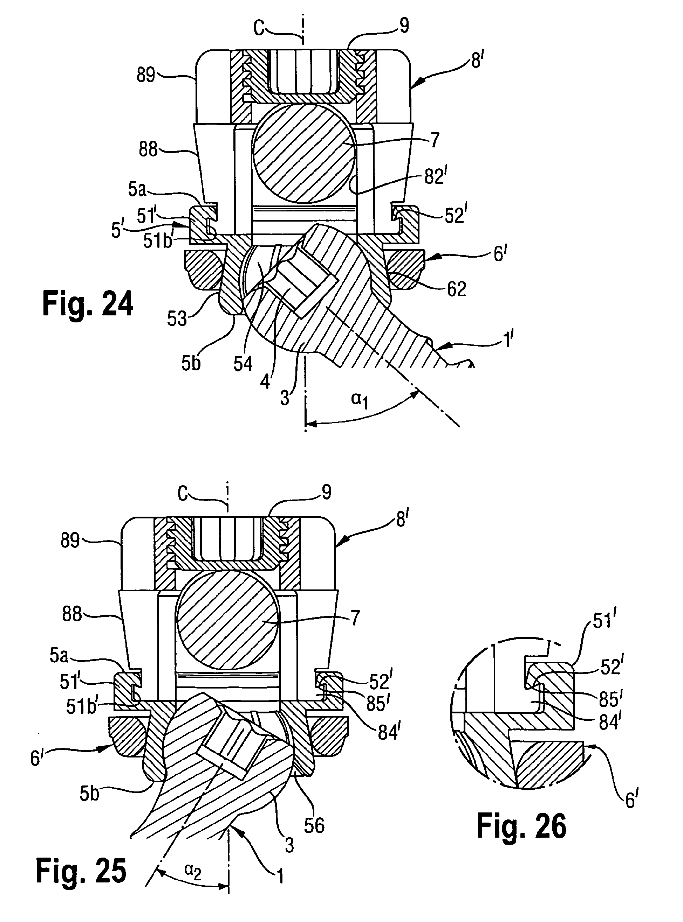

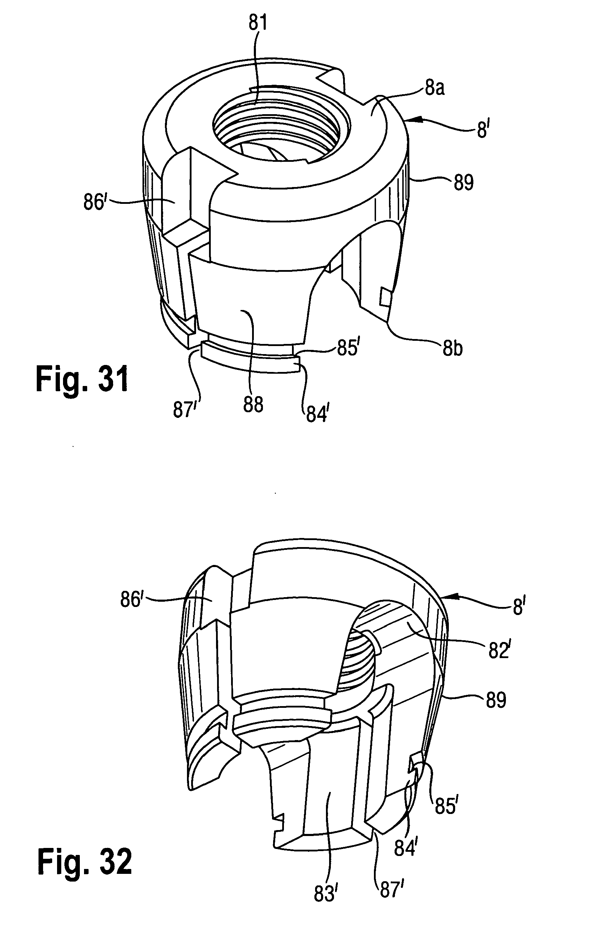

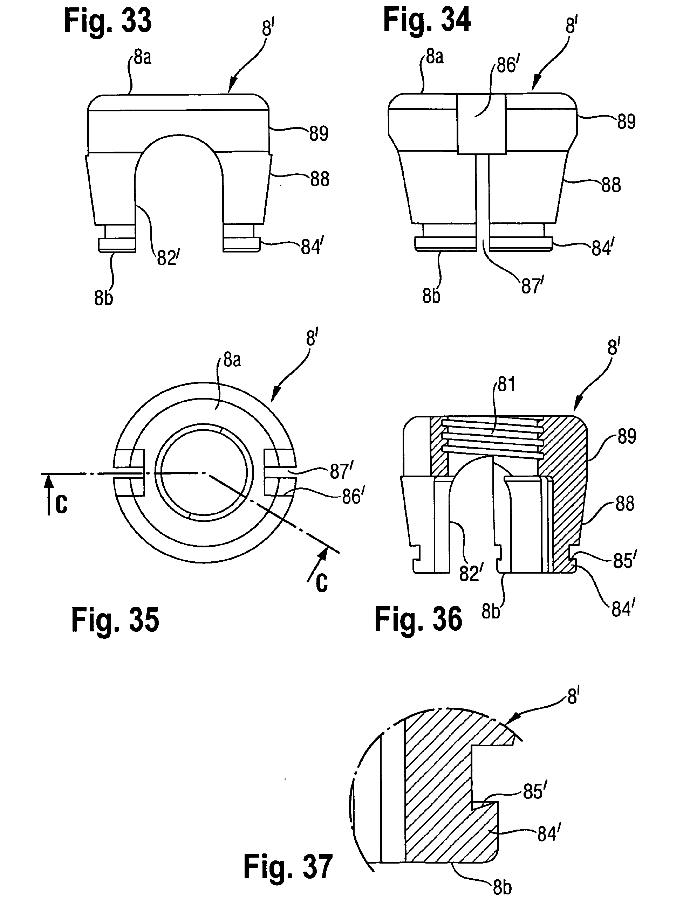

With reference to Figs. 30 to 37, the cap 8' has adjacent the second end 8b an outwardly projecting rim 84' with an undercut portion 85' with an inclined surface cooperating with undercut portion 52' of the head receiving part 5'. Furthermore, the cap 8' has a slightly conically-shaped outer surface portion 88 adjacent the second end and including the rim portion 84', the conical portion going over in a cylindrical portion 89. Inside, the cap has a cylindrical bore 83'. In the region of the conical outer surface portion 88, the wall thickness of the cap slightly decreases towards the second end 8b. The engagement portions 86' for a tool extend only into the cylindrical portion 89. Slits 87' extend completely through the conical outer surface portion 88 so as to render the cap flexible this region. Furthermore, the recess 82' receiving the rod is U-shaped. All other parts are the same as in the first embodiment. Assembly and use of the bone anchoring device according to the second embodiment similar to that the first embodiment with the difference that is shown in Figs. 24 to 26, the cap 8' is clicked from the inside into the groove 51b' of the head receiving part 5'.

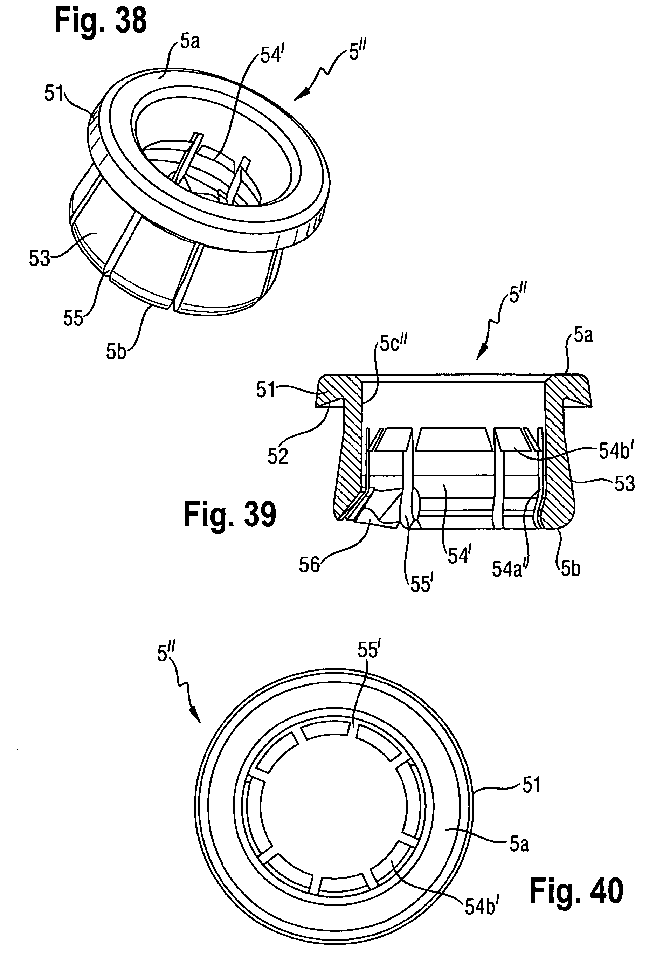

With reference to Figs. 38 to 40, third embodiment of the polyaxial bone anchoring device differs from the bone anchoring device according to the first embodiment in the design of the head receiving part. The head receiving part 5" has an axial through hole 5c" extending from the first and 5a in the direction of the second end 5b with a diameter larger than the diameter of the head 3 to allow the head 3 to pass therethrough. The hollow interior section 54' comprises a first spherically-shaped portion 54a' forming a seat for the head and a second narrowing portion 54b' at the side opposite to the second end 5b that narrows towards the first end 5a so that it forms an obstacle for the head. Slits 55' extend fully into the second portion 54b'. Hence, with the head receiving part 5" a top loading bone anchoring device is provided which allows to insert the bone anchoring element from the first end 5a and to pass it through the axial through hole 5c" thereby passing the narrowing portion which widens due to the slits until the head is seated in the spherical portion 54a'. Because of the narrowing portion 54b', the head 3 once it is inserted, is prevented from inadvertently being pushed out in the direction of the first and 5a.

Assembly and use is similar to that at of the first embodiment, the difference that the bone anchoring element is introduced from the first end 5a.

It shall be understood that also the second embodiment of the bone anchoring device maybe designed as a top loading bone anchoring device. For enabling this, the head receiving part has to be shaped like the head receiving part in the third embodiment with respect to the hollow interior portion for receiving the head.

Other modifications are conceivable. The configuration of the locking ring and the cooperating outer surface portion of the head receiving portion can be designed otherwise. For example, it is possible, that the inner surface of the locking ring is tapered and cooperates with the tapered outer surface of the head receiving part. Or, the outer surface portion of the head receiving part is convexly rounded and the inner surface of the locking ring is straight or tapered. The cooperating surfaces of locking ring and head receiving part can also be parallel so that the clamping of the head is achieved by an interference fit between the locking ring and the head receiving part.

For the bone anchoring element various bone anchoring elements can be used that differ with respect to their shank length, diameter or thread form or that have a through channel. Also, hooks, nails or any other anchoring elements are possible.

To generate the enlarged pivot angle, it is possible to use a symmetric head receiving portion that has been cut at the bottom in an inclined manner so as to generate an enlarged pivot angle or a larger circumferential area. The recessed area can also be achieved by a cut-out in a symmetrical head receiving part that provides an opening perpendicular to the central axis C.

The rod receiving recess can also be semi-circular, quarter-circular or U-shaped or can have any shape that is configured to accommodate a rod therein.

## Claims

1. A polyaxial bone anchoring device, the polyaxial bone anchoring device including
a bone anchoring element (1) having a shank (2) for anchoring in the bone and a head (3);
a head receiving part (5, 5', 5") having a first end (5a) and an open second end (5b) with a bounding edge and a hollow interior portion in communication with the open second end for receiving the head (3) therein, the head receiving part being flexible so as to allow clamping of the head;
a locking ring (6, 6') mounted around the head receiving part, the locking ring having a rod receiving portion (63, 64);
wherein the head (3) is pivotable in the head receiving part and can be locked at an angle by compressing the head receiving portion by means of the locking ring (8);
a cap (8, 8') configured to cover the rod and to engage the head receiving part,
a locking element extending through the cap and configured to engage the rod;
wherein the cap (8, 8") and the locking ring (6, 6') are rotatable with respect to the head receiving part (5, 5', 5") when the rod is inserted and upon tightening of the locking element the rod and the bone anchoring element are locked with respect to each other.

2. The bone anchoring device of claim 1, wherein the open end (5b) of the head receiving part has a bounding edge that is configured to permit the anchoring element (1) to pivot at a larger pivot angle at a first location of the bounding edge than at a second location of the bounding edge.

3. The polyaxial bone anchoring device of claim 1 or 2, wherein the bounding edge comprises a recessed area (56) that provides for an enlarged pivot angle when the bone anchoring element pivots into it.

4. The bone anchoring device of one of claims 1 to 3, wherein the head receiving part (5, 5', 5") comprises a first portion (51, 51') adjacent to the first end (5a) with an undercut (52, 52') for engagement by the cap.

5. The bone anchoring device of one of claims 1 to 4, wherein the head receiving part (5') has a groove (51b') for engagement by the cap.

6. The bone anchoring device of one of claims 1 to 5, wherein the head receiving part (5) comprises a second portion (53) with an outer surface that has a smallest outer diameter towards the first end (5a) with increasing outer diameter towards the open end (5b).

7. The bone anchoring device of one of claims 1 to 6, wherein the head receiving part (5) comprises a plurality of slits (55, 55') in its wall.

8. The bone anchoring device of one of claims 1 to 7, wherein the head (3) has a spherical outer surface portion and the head receiving part has a spherical seat portion (54, 54a') for the head.

9. The bone anchoring device of claim 8, wherein the head receiving part (5) has a through hole (5c) at the first end with a diameter smaller than a greatest diameter of the head (3).

10. The bone anchoring device of claim 8, wherein the head receiving part (5') has a through hole (5c") at the first end with a diameter greater than the diameter of the head (3) and wherein between the first end (5a) and the seat (54a') for the head a narrowing portion (54b') is provided that is expandable when the head is inserted through the first end (5a).

11. The bone anchoring device of one of claims 1 to 10, wherein the locking ring (6, 6') comprises two projections (63) opposite to each other in a circumferential direction, the projections each having a recess (64) configured to insert the rod and preliminarily hold the rod therein.

12. The bone anchoring device of claim 11, wherein the recess has a circle segment contour, the segment being preferably greater than a semi circle.

13. The bone anchoring device of one of claims 1 to 12, wherein the cap comprises a first end (8a) facing the head receiving part and a second end (8b) opposite thereto and two circumferentially opposite recesses (82, 82') for passing through the rod.

14. The bone anchoring device of one of claims 1 to 13, wherein the cap (8, 8') comprises a threaded through hole (81) and wherein the locking element (9) is a set screw provided in the through hole.

15. The bone anchoring device of one of claims 1 to 14, wherein the cap (8, 8') comprises at least one axial slit (87, 87') in its side wall rendering the cap flexible in radial direction.

16. The bone anchoring device of one of claims 1 to 15, wherein the cap (8') has a circumferential projection (84, 84') with an undercut portion (85, 85') for engagement with a portion (51,51') of the head receiving part.

17. A modular system of polyaxial bone anchoring devices, the system including
at least two bone anchoring elements (1) each having a shank (2) for anchoring in the bone and a head (3), wherein the bone anchoring elements differ in at least the design of the shank;
a head receiving part (5, 5', 5") having a first end (5a) and an open second end (5b) with a bounding edge and a hollow interior portion in communication with the open second end for receiving the head (3) therein, the head receiving part being flexible so as to allow clamping of the head;
a locking ring (6, 6') mounted around the head receiving part, the locking ring having a rod receiving portion (63, 64);
wherein the head (3) is pivotable in the head receiving part and can be locked at an angle by compressing the head receiving portion by means of the locking ring (8);
a cap (8, 8') configured to cover the rod and to engage the head receiving part,
a locking element extending through the cap and configured to engage the rod;
wherein the cap (8, 8") and the locking ring (6, 6') are rotatable with respect to the head receiving part (5, 5', 5") when the rod is inserted and upon tightening of the locking element the rod and the bone anchoring element are locked with respect to each other;
wherein the bone anchoring elements are selectively connectable to the head receiving part.

18. The system according to claim 17,
wherein , the open end (5b) of the head receiving part has a bounding edge that is configured to permit the anchoring element (1) to pivot at a larger pivot angle at a first location of the bounding edge than at a second location of the bounding edge; and wherein at least two head receiving parts are provided, the head receiving parts differing in the maximum pivot angle and wherein the head receiving parts are selectively connectable with one of the two bone anchoring elements.
